# EUROPÄISCHE PATENTSCHRIFT

(11) **EP 0 530 559 B1**
(45) Veröffentlichungstag und Bekanntmachung des Hinweises auf die Patenterteilung: **18.10.1995**
(21) Anmeldenummer: 92113802.0
(22) Anmeldetag: 13.08.1992
(51) Int. Cl.: C07C 205/61, C09B 1/00

(54) **Verfahren zur Herstellung von 1-Nitroanthrachinon-2-carbonsäuren**
Process for the preparation of 1-nitroanthraquinone-2-carboxylic acid
Procédé pour la préparation d'acides 1-nitroanthraquinone-2-carboxyliques

(30) Priorität: 27.08.1991 DE 4128348
(43) Veröffentlichungstag der Anmeldung: 10.03.1993
(73) Patentinhaber: BASF Aktiengesellschaft, 67063 Ludwigshafen (DE)
(72) Erfinder: Hagen, Helmut, Dr., W-6710 Frankenthal (DE); Dupuis, Jacques, Dr., W-6700 Ludwigshafen (DE); Acker, Michael, Dr., W-6900 Heidelberg (DE); Bergmann, Udo, Dr., W-6140 Bensheim (DE)

(56) Entgegenhaltungen:
- EP-A- 0 371 362
- DE-C- 229 394
- DATABASE WPI, Week 7749, Derwent Publications Ltd., London, GB; AN 77-87732Y; V. A. YAKOBI: "(1)-nitro or (1)-chloroanthrachinone-(2)-carboxylic acids preparation2, & SU-A-201 429

## Beschreibung

Die vorliegende Erfindung betrifft ein neues Verfahren zur Herstellung von 1-Nitroanthrachinon-2-carbonsäuren der allgemeinen Formel I 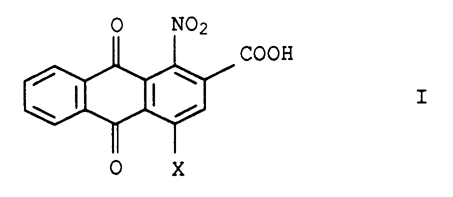
in der X Wasserstoff, Chlor oder Brom bedeutet, durch Oxidation eines 1-Nitro-2-methylanthrachinons der allgemeinen Formel II 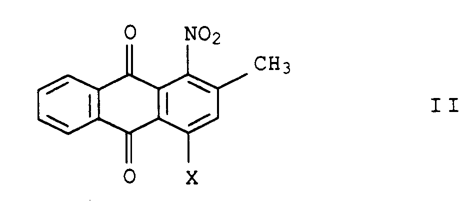
1-Nitroanthrachinon-2-carbonsäure und 1-Nitro-4-halogenanthrachinon-2-carbonsäuren sind wichtige Zwischenprodukte für die Herstellung von Küpenfarbstoffen wie Anthrachinon azolen, Acylaminoanthrachinonen und Phthaloylacridonen.

Sie sind bekanntermaßen auf verschiedene Weise zugänglich, und zwar meistens durch Oxidation der entsprechenden 2-Methylanthrachinone. Wie in den DE-A-2 29 394 und 22 42 643 beschrieben, werden hierbei Chrom(VI)salze in anorganischen Säuren als Oxidationsmittel eingesetzt. Bei diesen Verfahren ist jedoch von Nachteil, daß große Mengen an Chrom(III)salzen anfallen, die eine aufwendige Abwasseraufbereitung erforderlich machen.

Der Erfindung lag daher die Aufgabe zugrunde, 1-Nitroanthrachinon-2-carbonsäuren (I) in guten Ausbeuten und guten Reinheiten in verfahrenstechnisch einfacher und wirtschaftlicher Weise ohne Schwermetallbelastung des anfallenden Abwassers zugänglich zu machen.

Demgemäß wurde ein Verfahren zur Herstellung von 1-Nitroanthrachinon-2-carbonsäuren der allgemeinen Formel I 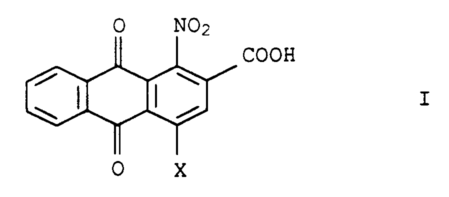
in der X Wasserstoff, Chlor oder Brom bedeutet, durch Oxidation eines 1-Nitro-2-methylanthrachinons der allgemeinen Formel II 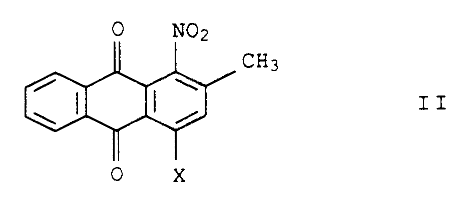
gefunden, welches dadurch gekennzeichnet ist, daß man die Oxidation in einem organischen Reaktionsmedium mit Salpetersäure als Oxidationsmittel vornimmt.

Die Konzentration der beim erfindungsgemäßen Verfahren eingesetzten Salpetersäure kann stark variiert werden, vorteilhaft sind jedoch Konzentrationen von 40 bis 100 Gew.-%, vor allem 50 bis 70 Gew.-%.

Für das erfindungsgemäße Verfahren geeignete organische Reaktionsmedien sind insbesondere bei der Reaktionstemperatur flüssige und nicht mit der Salpetersäure reagierende Benzolderivate. Geeignete Beispiele sind Nitrobenzol, Benzoesäure und deren Mischungen. Besonders bevorzugt ist dabei Nitrobenzol.

Die Lösungsmittelmenge ist frei wählbar, es empfiehlt sich jedoch, pro mol II 0,5 bis 2, vorzugsweise 1 bis 1,5 mol Lösungsmittel zu verwenden.

In der Regel führt man die Reaktion bei 180 bis 200°C, vorzugsweise bei 185 bis 190°C durch.

Üblicherweise nimmt man die Umsetzung bei Normaldruck vor, aber es ist auch möglich, unter Druck zu arbeiten.

Verfahrenstechnisch geht man vorzugsweise so vor, daß man das 1-Nitro-2-methylanthrachinon II in Nitrobenzol auf 180 bis 200°C erhitzt und bei dieser Temperatur die Salpetersäure zudosiert.

Zweckmäßigerweise führt man die Oxidation bis zu einem Umsatz von 50 bis 80 %, insbesondere etwa 70 % durch. Die Kontrolle kann in bekannter Weise chromatographisch erfolgen. Bei 70%igem Umsatz werden in der Regel 2 bis 6 mol, vorzugsweise 3 bis 5 mol Salpetersäure pro mol II benötigt.

Während der Umsetzung, die im allgemeinen 8 bis 12 Stunden in Anspruch nimmt, kann verdünnte Salpetersäure im Gemisch mit Nitrobenzol abdestilliert werden.

Man kann das erfindungsgemäße Verfahren sowohl diskontinuierlich als auch kontinuierlich durchführen.

Die Aufarbeitung des Reaktionsgemisches auf die 1-Nitroanthrachinon-2-carbonsäuren kann im Fall der Verwendung von Nitrobenzol als Lösungsmittel besonders vorteilhaft durch Abkühlen auf 100 bis 120°C, vorzugsweise auf 105 bis 110°C erfolgen. Bei dieser Temperatur fallen die Produkte I zum größten Teil aus und können durch Filtration abgetrennt werden, während der nicht umgesetzte Anteil der Ausgangsverbindungen II in der Mutterlauge gelöst bleibt und zur nächsten Oxidation wieder eingesetzt werden kann.

Zur Entfernung der Nitrobenzolreste aus den abfiltrierten Produkten I suspendiert man den noch feuchten Filterrückstand zweckmäßigerweise in Wasser und destilliert das Nitrobenzol mit Wasserdampf ab. Die Produkte I können dann durch Filtration aus der nitrobenzolfreien wäßrigen Suspension in so reiner Form isoliert werden, daß sie unmittelbar zu weiteren Zwecken wie vor allem der Farbstoffsynthese verwendet werden können.

Man erhält die 1- Nitroanthrachinon-2-carbonsäuren nach dem erfindungsgemäßen Verfahren auf verfahrenstechnisch einfache und wirtschaftliche Weise in guter Ausbeute und guter Reinheit.

Eine besonders bevorzugte Anwendung dieses Verfahrens besteht in der Herstellung der unsubstituierten 1-Nitroanthrachinon-2-carbonsäure.

### Beispiele

### Herstellung von 1-Nitroanthrachinon-2-carbonsäure

### Beispiel 1

Eine Mischung aus 1069 g (4,1 mol) 1-Nitro-2-methylanthrachinon und 4820 g Nitrobenzol wurde auf 190°C erhitzt. Bei dieser Temperatur wurden innerhalb von 10 h 4200 g (3000 ml; 43,3 mol) 65 gew.-%ige Salpetersäure und 2410 g (2000 ml) Nitrobenzol getrennt voneinander zudosiert (die eingesetzten Mengen entsprechen einem Umsatz von 70 %). In dieser Zeit wurden etwa 3000 ml 50 gew.-%ige Salpetersäure und etwa 2000 ml Nitrobenzol abdestilliert. Nach beendeter Salpetersäurezugabe wurde die Reaktionsmischung noch 30 min nachgerührt und anschließend auf 110°C abgekühlt.

Die dadurch ausgefällte 1-Nitroanthrachinon-2-carbonsäure wurde abfiltriert und in 4000 ml Wasser suspendiert. Nach Abdestillieren des noch im Feststoff enthaltenen Nitrobenzols mit Wasserdampf wurde die 1-Nitroanthrachinon-2-carbonsäure erneut durch Filtration aus der wäßrigen Suspension abgetrennt und bei 100°C getrocknet.

Es wurden 382 g 1-Nitroanthrachinon-2-carbonsäure mit einem Gehalt von > 98 % erhalten, was einer Ausbeute von 45 %, bezogen auf 70%igen Umsatz, entspricht.

### Beispiel 2

Eine Mischung aus der Nitrobenzolmutterlauge der Filtration in Beispiel 1 (enthaltend 1,2 mol nicht umgesetztes 1-Nitro-2-methylanthrachinon und nicht gefälltes Produkt) und 748 g (2,8 mol) neu eingesetztes 1-Nitro-2-methylanthrachinon wurde analog zu Beispiel 1 mit Salpetersäure umgesetzt.

Die weitere Aufarbeitung erfolgte ebenfalls analog zu Beispiel 1.

Es wurden 594 g 1-Nitroanthrachinon-2-carbonsäure mit einem Gehalt von > 98 % erhalten, was einer Ausbeute von 70 %, bezogen auf 70%igen Umsatz, entspricht.

## Patentansprüche

1. Verfahren zur Herstellung von 1-Nitroanthrachinon-2-carbonsäuren der allgemeinen Formel I 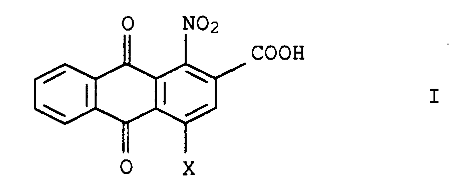 in der X Wasserstoff, Chlor oder Brom bedeutet, durch Oxidation eines 1-Nitro-2-methylanthrachinons der allgemeinen Formel II 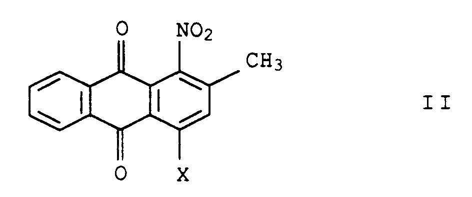 dadurch gekennzeichnet, daß man die Oxidation in einem organischen Reaktionsmedium mit Salpetersäure als Oxidationsmittel vornimmt.

2. Verfahren nach Anspruch 1, dadurch gekennzeichnet, daß man Nitrobenzol, Benzoesäure oder deren Mischungen als Reaktionsmedium verwendet.

3. Verfahren nach Anspruch 1 oder 2, dadurch gekennzeichnet, daß man 40 bis 100 gew.-%ige Salpetersäure als Oxidationsmittel einsetzt.

4. Verfahren nach den Ansprüchen 1 bis 3, dadurch gekennzeichnet, daß man die Oxidation bei 180 bis 200°C durchführt.

5. Verfahren nach den Ansprüchen 1 bis 4, dadurch gekennzeichnet, daß man bis zu einem Umsatz von 50 bis 80 % oxidiert.

6. Verfahren nach den Ansprüchen 1 bis 5, dadurch gekennzeichnet, daß man es zur Herstellung von 1-Nitroanthrachinon-2-carbonsäure anwendet.

## Claims

1. A process for preparing 1-nitroanthraquinone-2-carboxylic acids of the general formula I 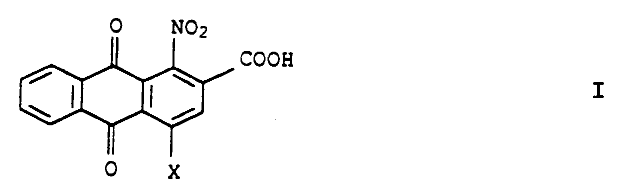 where X is hydrogen, chlorine or bromine, by oxidizing a 1-nitro-2-methylanthraquinone of the general formula II 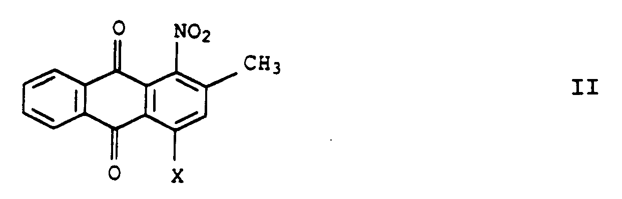 which comprises carrying out the oxidation in an organic reaction medium using nitric acid as oxidizing agent.

2. A process as claimed in claim 1, wherein the reaction medium used comprises nitrobenzene, benzoic acid or a mixture thereof.

3. A process as claimed in claim 1 or 2, wherein the oxidizing agent used comprises 40 - 100 % strength by weight nitric acid.

4. A process as claimed in any of claims 1 to 3, wherein the oxidation is carried out at from 180 to 200°C.

5. A process as claimed in any of claims 1 to 4, wherein the oxidation is carried on to a conversion of from 50 to 80 %.

6. A process as claimed in any of claims 1 to 5 employed for preparing 1-nitroanthraquinone-2-carboxylic acid.

## Revendications

1. Procédé de préparation d'acides 1-nitroanthraquinone-2-carboxyliques de formule générale I 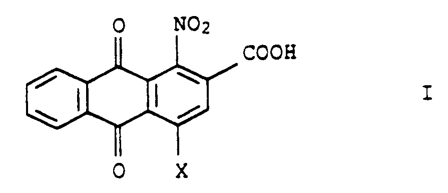 dans laquelle X représente un atome d'hydrogène, de chlore ou de brome, par oxydation d'une 1-nitro-2-méthylanthraquinone de formule générale II 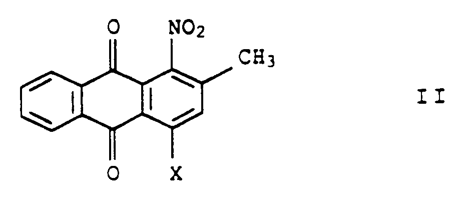 caractérise en ce que l'on procède à l'oxydation dans un milieu réactionnel organique, avec de l'acide nitrique comme oxydant.

2. Procédé selon la revendication 1, caractérisé en ce que l'on utilise, comme milieu réactionnel, du nitrobenzène, de l'acide benzoïque ou des mélanges de ceux-ci.

3. Procédé selon la revendication 1 ou 2, caractérisé en ce que l'on utilise de l'acide nitrique à 40-100% en poids comme oxydant.

4. Procédé selon l'une quelconque des revendications 1 à 3, caractérisé en ce que l'on conduit l'oxydation à une température de 180 à 200°C.

5. Procédé selon l'une quelconque des revendications 1 à 4, caractérisé en ce que l'on oxyde jusqu'à un degré de transformation de 50 à 80%.

6. Procédé selon l'une quelconque des revendications 1 à 5, caractérisé en ce qu'on l'applique pour la préparation d'acide 1-nitroanthraquinone-2-carboxylique.
